# EUROPÄISCHE PATENTANMELDUNG

(11) **EP 4 487 759 A1**
(43) Veröffentlichungstag der Anmeldung: **08.01.2025**
(21) Anmeldenummer: 23183663.6
(22) Anmeldetag: 05.07.2023
(51) Int. Cl.: A61B 5/00, A61B 1/00, A61B 1/07, A61B 1/313, A61B 10/02, A61B 17/00, A61B 18/00, A61B 5/06

(54) **MEDIZINISCHE EINGRIFFSANORDNUNG UND COMPUTERIMPLEMENTIERTES VERFAHREN ZUR ERMITTLUNG EINER POSITIONSINFORMATION EINES MEDIZINISCHEN INSTRUMENTS**

(71) Anmelder: Siemens Healthineers AG, 91301 Forchheim (DE)
(72) Erfinder: Kellnberger, Stephan, 91054 Erlangen (DE); Hornung, Oliver, 91364 Unterleinleiter (DE)
(74) Vertreter: Siemens Healthineers Patent Attorneys

(57) **Zusammenfassung**

Die Erfindung betrifft eine medizinische Eingriffsanordnung (1), umfassend
- ein längliches medizinisches Instrument (2) zum teilweisen Einführen in ein Eingriffsgebiet (39) eines Untersuchungsobjekts (5), wobei innerhalb des medizinischen Instruments (2) wenigstens ein Lichtleiter (6) zu wenigstens einer distalen Messposition (36, 52), in der der Lichtleiter (6) zum Senden und Empfangen von Licht offenliegt, geführt ist, und
- ein extern zum Untersuchungsobjekt (5) angeordnetes Messgerät (9), das mit dem proximalen Ende des wenigstens einen Lichtleiters (6) verbunden ist und aufweist:
- eine Lichtquelle (8) zur Erzeugung von durch wenigstens einen des wenigstens einen Lichtleiters (6) zu der wenigstens einen Messposition (36, 52) zu führendem Sendelicht eines Sendespektrums,
- einen Lichtdetektor (10) zum Empfang von Empfangslicht, das von der wenigstens einen Messposition (36, 52) über wenigstens einen des wenigstens einen Lichtleiters (6) zu dem Messgerät (9) geführt ist, und
- eine Auswertungseinheit (16) zur Auswertung von das Empfangsspektrum des Empfangslichts beschreibenden Messdaten in Kenntnis des Sendespektrums zur Ermittlung einer die Position des medizinischen Instruments (2) in dem Untersuchungsobjekt (5) beschreibenden Positionsinformation auf der Grundlage von Reflektionseigenschaften von Materialien, insbesondere Geweben, des Untersuchungsobjekts (5);
und

- eine Darstellungseinrichtung (17) zur Ausgabe der Positionsinformation.

## Beschreibung

Die Erfindung betrifft eine medizinische Eingriffsanordnung, umfassend ein längliches medizinisches Instrument, insbesondere eine Nadel, zum teilweisen Einführen in ein Eingriffsgebiet eines Untersuchungsobjekts. Daneben betrifft die Erfindung ein computerimplementiertes Verfahren zur Ermittlung einer Positionsinformation eines in einem Eingriffsgebiet eines Untersuchungsobjekt befindlichen länglichen medizinischen Instruments, insbesondere einer Nadel.

Im Stand der Technik wurden bereits verschiedene Arten minimalinvasiver Eingriffe an einem Untersuchungsobjekt, insbesondere einem Patienten, vorgeschlagen. Minimalinvasive Eingriffe haben den Vorteil einer möglichst geringen Belastung des Patienten. Hierbei wird ein medizinisches Instrument wenigstens teilweise in ein Eingriffsgebiet in einem Patienten eingeführt, wobei das medizinische Instrument, insbesondere an der Instrumentenspitze, also dem distalen Ende, üblicherweise ein Eingriffsmittel, insbesondere ein Untersuchungs- und/oder Behandlungsmittel, aufweist. Das medizinische Instrument wird bis an eine Zielposition bzw. in einen Zielbereich vorgeschoben, an dem der Eingriff stattfinden soll.

Ein Beispiel für derartige medizinische Instrumente sind Nadeln, beispielsweise Biopsienadeln, mit denen eine Gewebeprobe entnommen werden kann, und/oder Behandlungsdaten zur Behandlung des Zielgewebes im Zielbereich. Behandlungsnadeln können hierbei insbesondere Ablationsnadeln umfassen, mit denen krankhaftes Gewebe, insbesondere Tumorgewebe, behandelt, insbesondere verödet, werden kann. Beispielsweise wurden als Behandlungsmittel Energieemitter an der Spitze des Instruments vorgeschlagen, deren abgegebene Energie Gewebeschäden, beispielsweise durch Überhitzung des Gewebes, erzeugt und somit krankhaftes Gewebe, beispielsweise Tumoren, zerstört. Derartige Eingriffe sind sowohl für Weichgewebe, insbesondere in Organen wie der Leber, als auch für muskuloskelettale Anwendungen, beispielsweise Wirbelsäulentumoren, bekannt.

Bei diesen minimalinvasiven Eingriffen, insbesondere aber bei der Behandlung von Zielgewebe, ist es äußerst wichtig, die Position des medizinischen Instruments zu überprüfen und das Erreichen des Zielbereichs bzw. des Zielgewebes sicherzustellen. Hierzu wurden im Stand der Technik bereits verschiedene Ansätze vorgeschlagen.

Zunächst ist es bekannt, die Positionierung des medizinischen Instruments bildbasiert zu überwachen. Als Bildgebungmodalität wird dabei üblicherweise die Computertomografie eingesetzt, wobei in einem "Step-and-Shoot"-Verfahren mehrere Schichtbilder während des Einschubs aufgenommen werden, um die Überwachung vorzunehmen. Dies erlaubt allerdings keine Echtzeit-Überwachung, sondern ein schrittweises Vorgehen, bei dem die den Eingriff durchführende Person für jeden Aufnahmevorgang den Raum verlassen muss. Für bestimmte Arten von Eingriffen, insbesondere in der Leber, wurde auch vorgeschlagen, Ultraschallbildgebung zur Überwachung der Positionierung des Instruments zu verwenden, wobei beispielsweise Tumoren in Leber-Parenchym nicht mit hinreichend hohem Kontrast dargestellt werden und zudem aufgrund der tieferen Lage bei adipösen Patienten die Bildqualität nachlässt. Auch Knochenstrukturen senken hier die Bildqualität.

Zur Unterstützung der den Eingriff durchführenden Person wurde ferner vorgeschlagen, den Instrumentenpfad vorab, beispielsweise anhand eines dreidimensionalen Planungs-Bilddatensatzes, zu planen und eine Lichtführungseinrichtung, insbesondere eine Laserführungseinrichtung, zu verwenden, wobei beispielsweise das medizinische Instrument in seiner Längsrichtung anhand eines Laserkreuzes ausgerichtet werden kann. Auch der Eintrittspunkt kann neben der Angulation projiziert werden, in diesem Fall auf die Oberfläche des Patienten. Allerdings ist auch bei einer solchen Unterstützung noch eine Überwachung hinsichtlich der Vorschubstrecke innerhalb des Patienten erforderlich, was bei derartigen Systemen häufig mittels Bildgebung geschieht.

Daher gestaltet sich eine verlässliche Echtzeit-Überwachung beim Vorschub von medizinischen Instrumenten im Eingriffsgebiet des Patienten bislang schwierig, sodass ein Echtzeit-Feedback, insbesondere bezüglich des Erreichens des Zielbereichs und auf automatisierte Weise, nicht möglich ist.

Der Erfindung liegt daher die Aufgabe zugrunde, ein Verfahren zum Bereitstellen von Feedback zum Vorschub eines medizinischen Instruments in Echtzeit anzugeben.

Aufgabe wird erfindungsgemäß gelöst durch eine Eingriffsanordnung mit den Merkmalen des Anspruchs 1 und ein Verfahren mit den Merkmalen des Anspruchs 15. Vorteilhafte Weiterbildungen ergeben sich aus den Unteransprüchen.

Eine erfindungsgemäße medizinische Eingriffsanordnung weist auf:
- ein längliches medizinisches Instrument, insbesondere eine Nadel, zum teilweisen Einführen in ein Eingriffsgebiet eines Untersuchungsobjekts, wobei innerhalb des medizinischen Instruments wenigstens ein Lichtleiter zu wenigstens einer distalen Messposition, in der der Lichtleiter zum Senden und Empfangen von Licht offenliegt, geführt ist, und
- ein extern zum Untersuchungsobjekt angeordnetes Messgerät, das mit dem proximalen Ende des wenigstens einen Lichtleiters verbunden ist und aufweist:
   - eine Lichtquelle zur Erzeugung von durch wenigstens einen des wenigstens einen Lichtleiters zu der wenigstens einen Messposition zu führendem Sendelicht eines Sendespektrums,
   - einen Lichtdetektor zum Empfang von Empfangslicht, das von der wenigstens einen Messposition über wenigstens einen des wenigstens einen Lichtleiters zu dem Messgerät geführt ist, und
   - eine Auswertungseinheit zur Auswertung von das Empfangsspektrum des Empfangslichts beschreibenden Messdaten in Kenntnis des Sendespektrums zur Ermittlung einer die Position des medizinischen Instruments in dem Untersuchungsobjekt beschreibenden Positionsinformation auf der Grundlage von Reflektionseigenschaften von Materialien, insbesondere Geweben, des Untersuchungsobjekts;
   und
- eine Darstellungseinrichtung zur Ausgabe der Positionsinformation.

Dabei ist die Positionsinformation im Rahmen der vorliegenden Erfindung nicht zwangsläufig als eine exakte Positionsangabe zu verstehen, sondern eine Position ist beispielsweise auch angegeben, wenn eine Materialklasse des Materials an wenigstens einer Messposition, insbesondere ein Verlauf von Materialklassen entlang des distalen, in das Untersuchungsobjekt eingebrachten Anteils des medizinischen Instruments, bekannt ist und/oder ermittelt wird, ob bzw. welche Messposition bzw. Messpositionen innerhalb des Untersuchungsobjekts befindlich sind.

Erfindungsgemäß wird mithin vorgeschlagen, mittels eines Lichtleiters, insbesondere wenigstens einer optischen Faser, die Reflektionseigenschaften von Gewebe an der wenigstens einen Messposition zu nutzen, um die Positionsinformation herzuleiten. Dabei sollen Reflektionseigenschaften nicht nur die gerichtete Reflektion, sondern auch Rückstreueigenschaften, also die diffuse Reflektion, beschreiben. Dies entspricht der üblichen Aufteilung in Transmission, Absorption und Reflektion. Reflektionseigenschaften können dabei beispielsweise einen Reflektionskoeffizienten und/oder einen Reflektionsgrad umfassen bzw. durch diese Parameter beschrieben sein.

Der Lichtleiter verläuft dabei im und/oder außen am länglichen medizinischen Instrument, insbesondere der Nadel. Er ist somit integriert und kann während der Nutzung des medizinischen Instruments zur Ermittlung von Echtzeit-Informationen, hier den Positionsinformationen, eingesetzt werden. Es wird also eine Echtzeit-Detektion der Instrumentenprogression im Untersuchungsobjekt für minimalinvasive Prozeduren bereitgestellt.

Hierbei kann mit besonderem Vorteil eine Kombination mit einer Laserführung, generell eine Lichtführungseinrichtung, die basierend auf einer Planung den Eintrittspunkt und die korrekte Vorschuborientierung des medizinischen Instruments anzeigen kann, vorgenommen werden, da die erfindungsgemäße Vorgehensweise letztlich ideal ergänzt, nämlich die bei der Unterstützung der den Eingriff durchführenden Person noch fehlende Positionsinformation hinsichtlich der Vorschubstrecke hinzufügt. Insbesondere kann daher vorgesehen sein, dass die Eingriffsanordnung zusätzlich eine Lichtführungseinrichtung, insbesondere eine Laserführungseinrichtung, zur Markierung eines Eintrittspunktes und/oder einer Sollorientierung des medizinischen Instruments gemäß einer Planungsinformation aufweist. So werden die bereits hervorragenden Unterstützungseigenschaften durch die Laserführung um eine Echtzeit-Detektion der (zumindest groben) Vorschubstrecke ergänzt.

Dabei basiert die Erfindung, generell gesprochen, auf der Idee, dass Veränderungen an der wenigstens einen Messposition, insbesondere beim Eintritt in das Untersuchungsobjekt, bevorzugt aber auch bei Materialgrenzen innerhalb des Untersuchungsobjekts, klare Hinweise auf den Ort der entsprechenden Messposition geben. Dies kann auf verschiedene Weise konkret genutzt werden, worauf im Folgenden hinsichtlich der konkreten Ausführungsbeispiele der vorliegenden Erfindung noch genauer eingegangen werden wird. Konkret kann die Positionsinformation vorzugsweise beschreiben, wie weit, zumindest approximiert, das medizinische Instrument in das Untersuchungsobjekt eingeschoben ist und/oder in welcher Gewebeart/welchem Gewebetyp oder allgemein Materialart/Materialtyp sich die Spitze, also das distale Ende, des Instruments befindet.

Auf diese Weise wird die Nutzung von Röntgenbildgebung, insbesondere Computertomografie-Bildgebung, zweckmäßigerweise stark reduziert, sodass das Untersuchungsobjekt geringeren Röntgendosen ausgesetzt wird. Auf diese Weise wird, nachdem die "Step-and-Shoot"-Vorgehensweise deutlich reduziert oder gänzlich unnötig wird, auch die für einen Eingriff benötigte Zeit reduziert. Weiterhin ist vorteilhaft, dass der Lichtleiter, insbesondere als wenigstens eine optische Faser, leicht in medizinische Instrumente, beispielsweise eine Ablationsnadel, integriert werden kann.

Allgemein gesprochen kann die Eingriffsanordnung im Übrigen auch eine Steuereinrichtung umfassen, die in dem Messgerät und/oder extern zu diesem verbaut sein kann und den Betrieb der Eingriffsanordnung insgesamt steuert. Beispielsweise kann die Steuereinrichtung eine Ausgabeeinheit zur Ausgabe der Positionsinformation an der Darstellungseinrichtung umfassen. Umfasst die Eingriffsanordnung auch eine Bildgebungseinrichtung, kann auch diese, insbesondere im Kontext des Eingriffs, mittels einer Aufnahmeeinheit der Steuereinrichtung angesteuert werden, beispielsweise zur Aufnahme des Planungs-Bilddatensatzes und/oder für einen Kontrollscan. Auch kann die Steuereinrichtung eine Planungseinheit zur Ermittlung einer einen geplanten Instrumentenpfad beschreibenden Planungsinformation aufweisen. Ist auch eine Lichtführungseinrichtung als Teil der Eingriffsanordnung vorgesehen, kann die Steuereinrichtung auch eine Ansteuereinheit zur Ansteuerung der Lichtführungseinrichtung zur Markierung eines Eintrittspunktes gemäß der Planungsinformation und/oder zur Markierung einer Orientierung des medizinischen Instruments gemäß dem Instrumentenpfad der Planungsinformation umfassen. Die Ansteuereinheit kann auch weitere Komponenten ansteuern.

Bei dem Untersuchungsobjekt kann es sich um einen menschlichen oder tierischen Patienten oder eine menschliche oder tierische Patientin und/oder ein Eingriffsphantom handeln. Bei dem Instrument handelt es sich bevorzugt um eine Nadel, insbesondere eine Ablationsnadel oder sonstige Behandlungsnadel, bevorzugt für Tumoren. Es kann sich bei dem medizinischen Instrument jedoch auch um ein anderes minimalinvasives Instrument handeln, beispielsweise einen Katheter oder dergleichen.

In zweckmäßigen Ausführungsbeispielen kann vorgesehen sein, dass die Auswertungseinheit ausgebildet ist, bei der Ermittlung der Positionsinformation einen über den Vorschub in das Untersuchungsobjekt entstehenden Verlauf der Messdaten zu verwenden. Wird der Verlauf betrachtet, welcher insbesondere verschiedene Materialklassengrenzen und/oder Intensitätsänderungen, beispielsweise durch zusätzlich in das Untersuchungsobjekt eintretende Messpositionen, entlang des Vorschubs beschreiben kann, wird, allgemein gesagt, eine Zusatzinformation genutzt, die eine genauere und robustere Ermittlung der Positionsinformation erlaubt. Konkrete Ausführungsbeispiele, die den Verlauf der Messdaten nutzen, werden im Folgenden noch genauer erläutert.

Insbesondere für Ausführungsbeispiele, in denen aus den durch die Messdaten abgebildeten Reflektionseigenschaften auf Materialklassen, insbesondere Gewebeklassen, gefolgert wird, spielen unterschiedliche Reflektionsverhalten von unterschiedlichen Materialien eine Rolle. Reflektionseigenschaften sind oft wellenlängenabhängig, so dass zweckmäßige Ausgestaltungen der vorliegenden Erfindung vorsehen, dass das Sendespektrum mehrere Wellenlängen und/oder Wellenlängenbereiche abdeckt. Beispielsweise können Kombinationen von Wellenlängen und/oder Wellenlängenbereichen gewählt werden, die kombinatorisch unter Kenntnis der vorkommenden Materialien/Materialklassen die gewünschten Unterscheidungen möglichst gut erlauben. So zeigen beispielsweise sauerstoffreiches und sauerstoffarmes Blut im Bereich um 700 nm deutlich unterschiedliche Reflektionseigenschaften. Untersuchungen zu optischen Eigenschaften unterschiedlicher Materialien, insbesondere von Geweben, finden sich im Stand der Technik und können auch im Rahmen der vorliegenden Erfindung verwendet werden.

Insgesamt kann also gesagt werden, dass vorteilhafter Weise nicht nur eine Wellenlänge im Sendespektrum eingesetzt wird, sondern mehrere diskrete Wellenlängen oder Wellenlängenbereiche, insbesondere auch ein breites Sendespektrum, verwendet werden können, um eine multispektrale Messung zu erlauben. Dies erhöht weiter die Genauigkeit und Robustheit der Messung.

Allgemein kann bevorzugt das Spektrum im optischen und/oder nahinfraroten Wellenlängenbereich liegen. Bei diesen Frequenzen zeigen sich hinsichtlich der Reflektionseigenschaften geeignete Unterschiede, wobei zudem die Messung für einen Patienten gut verträglich ist.

Während es grundsätzlich denkbar ist, worauf für Ausführungsbeispiele noch näher eingegangen wird, unabhängig von den jeweiligen Materialien, insbesondere Geweben, zu arbeiten, sieht eine zweckmäßige Weiterbildung vor, dass die Auswertungseinheit ausgebildet ist, als Positionsinformation und/oder bei der Ermittlung der Positionsinformation aus den Messdaten wenigstens eine Materialklasse, insbesondere Gewebeklasse, des an wenigstens einer der wenigstens einen Messposition das Sendelicht in das Empfangslicht reflektierenden Materials und/oder einen Übergang zwischen solchen Materialklassen, insbesondere Gewebeklassen, zu ermitteln. Wie bereits erwähnt, stellt bereits die Angabe einer Gewebeklasse, an der sich die wenigstens eine Messposition befindet, eine zweckmäßige Positionsinformation dar, beispielsweise wenn die Messposition mit einem Behandlungsmittel zusammenfällt, da dann festgestellt werden kann, ob das korrekte Gewebe erreicht ist. Anders gesagt kann vorgesehen sein, dass die Positionsinformation eine Anordnung wenigstens einer der wenigstens einen Messposition in einem Materialabschnitt, insbesondere Gewebeabschnitt, des Untersuchungsobjekts beschreibt. Doch lässt sich die Materialklasse auch als Zwischenergebnis auf dem Weg zu einer Positionsinformation verwenden, insbesondere wenn Hintergrundwissen zu dem Eingriffsgebiet vorliegt, das eine konkretere Lage von Materialabschnitten, insbesondere Gewebeabschnitten, einer Materialklasse in dem Eingriffsgebiet beschreibt. Ist durch das Hintergrundwissen bekannt, wo sich bestimmte Materialklassen und Grenzen der Materialklassen innerhalb des Eingriffsgebiets befinden, können durch Kenntnis der Materialklassen unter Hinzunahme des Hintergrundwissens, beispielsweise in Form von (gegebenenfalls annotierten) Bilddaten hieraus zumindest Anteile der Positionsinformation hergeleitet werden, beispielsweise auch konkrete Materialabschnitte, insbesondere Gewebeabschnitte, in denen sich die Messposition und somit das Instrument befindet.

Hierbei wird ausgenutzt, dass, wie dargestellt, die zurückreflektierte Intensität mit unterschiedlichen Materialklassen, insbesondere Gewebeklassen, gemäß deren Reflektionseigenschaften korreliert werden kann. Ist beispielsweise der Reflektionskoeffizient verschiedener Materialklassen, insbesondere verschiedener Gewebeklassen, als Funktion der Wellenlänge bekannt, kann unter Kenntnis des Sendespektrums aus dem Empfangsspektrum gefolgert werden, welches Material bzw. Gewebe an der Messposition vorliegt. Anders gesagt kann eine Klassifizierung vorgenommen werden. Beispielsweise haben für 750 nm sauerstoffführendes und nicht sauerstoffführendes Hämoglobin unterschiedliche Reflektionseigenschaften, die eine Unterscheidung erlauben. Ein ähnliches Verhalten kann für andere Gewebeklassen, beispielsweise Muskelgewebe und Fettgewebe, beobachtet werden. Mithin erlaubt die Messung des Empfangslichts und die Beurteilung des entsprechenden Empfangsspektrums, insbesondere wellenlängenspezifischer Intensitäten, unter Kenntnis des Sendespektrums eine Klassifizierung des Materials am Ort der Messposition, an der das Empfangslicht zurückreflektiert wurde.

In einer besonders bevorzugten Weiterbildung der Erfindung kann in diesem Zusammenhang vorgesehen sein, dass die Auswertungseinheit zur Berücksichtigung von Bilddaten des Eingriffsgebiets des Untersuchungsobjekts bei der Klassifizierung und/oder zur Zuordnung wenigstens einer der wenigstens einen Messposition zu einem bzw. dem in den Bilddaten sichtbaren Materialabschnitt ausgebildet ist. Materialabschnitte sind hierbei solche Abschnitte beim Vorschub des medizinischen Instruments, die eine bestimmte Materialklasse umfassen. In diesem Zusammenhang ist es besonders zweckmäßig, auch den Verlauf der Messdaten und somit der Empfangsspektren mit dem Vorschub zu berücksichtigen, da die aus den Bilddaten bekannten Materialabschnitte beim Einführen des medizinischen Instruments in das Untersuchungsobjekt in der in den Bilddaten dargestellten Reihenfolge durchlaufen werden. Hierbei zeigt jeder Übergang zwischen Materialklassen, also jede entsprechende Veränderung im Empfangsspektrum, einen Materialabschnittswechsel an. Sind die Bilddaten annotiert oder kann anderweitig die dargestellte Materialklasse hergeleitet werden, kann auch allein aus den Bilddaten und der sich daraus ergebenden, zwangsläufig zu durchlaufenden Reihenfolge von Materialklassen in Materialabschnitten, gefolgert werden, in welchem Materialabschnitt welcher Materialklasse sich die Messposition nach Durchlaufen einer entsprechenden Anzahl von Materialübergängen befinden muss. Insbesondere kann hierbei bei Bestimmung einer Materialklasse aus dem Empfangsspektrum auch eine Plausibilisierung erfolgen. Mit besonderem Vorteil dienen die Bilddaten aber in jedem Fall dazu, eine räumliche Zuordnung zu erlauben, da die räumliche Lage der Materialabschnitte in den Bilddaten ja bekannt ist, insbesondere dann, wenn die Bildgebungseinrichtung, mit der sie aufgenommen wurden, Teil der Eingriffsanordnung ist oder zumindest eine Registrierung stattfinden konnte. In Ausführungsbeispielen ist es also denkbar, Materialklassen an einer Messposition zu nutzen, um die Befindlichkeit einer entsprechenden Messposition in einem Materialabschnitt dieser Materialklasse als Positionsinformation zu bestimmen, wobei bei Eintritt in den Materialabschnitt die genaueste Positionsbestimmung vorliegt.

Dabei können die Bilddaten beispielsweise einen Vorab-Bilddatensatz, insbesondere einen Planungs-Bilddatensatz, und/oder Bilddaten eines Kontrollscans und/oder einer Live-Bildgebung, umfassen. Vorab-Bilddaten und während des Eingriffs aufgenommene Bilddaten können zweckmäßig miteinander registriert sein oder werden. Insbesondere im Fall des Vorab-Bilddatensatzes, beispielsweise des Planungs-Bilddatensatzes, können die Bilddaten auch annotiert sein, wie bereits kurz dargelegt.

In einem konkreten Beispiel einer Messposition und der Ablation eines Lebertumors wird die Messposition beim Einschieben des medizinischen Instruments in das Untersuchungsobjekt, insbesondere den Patienten, folgende Materialübergänge bzw. Materialien passieren: Haut, Fett, Blut, Leberkapsel, Leber-Parenchym und Tumor. Wann immer sich das Empfangsspektrum verändert, kann gefolgert werden, dass in einen neuen Materialabschnitt eingetreten wird, sodass insbesondere mit Bezug auf die Bilddaten jederzeit leicht die Position des medizinischen Instruments/der Messposition nachverfolgt werden kann. Ähnliches ist auch bei muskuloskelettalen Anwendungen möglich, wenn beispielsweise Haut, Fett, und Knochen passiert werden, bevor ein Tumor oder sonstiges krankhaftes Gewebe erreicht wird.

Vorzugsweise kann bei der Nutzung von Bilddaten die Eingriffsanordnung, insbesondere als Teil der Steuereinrichtung, ferner eine bzw. die Ausgabeeinheit aufweisen, welche zur Ausgabe einer auf den Bilddaten basierenden, die Positionsinformation anzeigenden Darstellung ausgebildet ist. Beispielsweise kann in den Bilddaten der Materialabschnitt, insbesondere Gewebeabschnitt, hervorgehoben werden, in dem sich die Messposition, beispielsweise an der Spitze des Instruments, bzw. das Instrument allgemein gerade befindet. Insbesondere in Kombination mit einer Lichtführungseinrichtung, die Planungsinformationen, die auf einem Planungs-Bilddatensatz basieren, nutzt, stellt dies eine einfache und intuitive Fortsetzung der Unterstützung hinsichtlich Eintrittspunkt und Orientierung dar, wobei insbesondere Planungs-Bilddaten und zusätzlich überlagerte Planungs-Informationen als Grundlage der Darstellung herangezogen werden können.

Vorzugsweise kann die Auswertungseinheit zur Verwendung einer trainierten Funktion zur Klassifizierung ausgebildet sein. Es kann also auch im Rahmen der vorliegenden Erfindung auf Künstlicher Intelligenz basierende Mustererkennung/Materialklassifikation, insbesondere Gewebeklassifikation, eingesetzt werden, wobei zum Trainieren einer solchen trainierten Funktion beispielsweise mit dem Instrument bei in einer bekannten Materialklasse angeordneter Messposition Trainings-Messdaten bzw. Empfangsspektren aufgenommen werden können und zum Anlernen der trainierten Funktion verwendet werden können. Hierbei können die Trainings-Messdaten insbesondere auch Übergänge zwischen verschiedenen Materialklassen umfassend aufgenommen werden.

Im Allgemeinen bildet eine trainierte Funktion kognitive Funktionen ab, die Menschen mit anderen menschlichen Gehirnen assoziieren. Durch Training basierend auf Trainingsdaten (Maschinenlernen) ist die trainierte Funktion in der Lage, sich an neue Umstände anzupassen und Muster zu detektieren und zu extrapolieren.

Allgemein gesagt können Parameter einer trainierten Funktion durch Training angepasst werden. Insbesondere können überwachtes Lernen, halbüberwachtes Lernen, nicht überwachtes Lernen, Reinforcement Learning und/oder aktives Lernen verwendet werden. Darüber hinaus kann auch Repräsentationslernen (auch als "feature learning" bekannt) eingesetzt werden. Die Parameter der trainierten Funktion können insbesondere iterativ durch mehrere Trainingsschritte angepasst werden.

Eine trainierte Funktion kann beispielsweise ein neuronales Netz, eine Support Vector Machine (SVM), einen Entscheidungsbaum und/oder ein Bayes-Netzwerk umfassen und/oder die trainierte Funktion kann auf k-means-Clustering, Q-Learning, genetischen Algorithmen und/oder Zuordnungsregeln basieren. Insbesondere kann ein neuronales Netzwerk ein tiefes neuronales Netzwerk, ein Convolutional Neural Network (CNN) oder ein tiefes CNN sein. Darüber hinaus kann das neuronale Netzwerk ein Adversarial Network, ein tiefes Adversarial Network und/oder ein Generative Adversarial Network (GAN) sein.

Alternativ oder zusätzlich können auch durch klassische Auswertung die Reflektion, bevorzugt wellenlängenabhängig, kennzeichnende Parameter, beispielsweise Reflektionskoeffizienten und dergleichen, ermittelt werden und mit entsprechenden Materialeigenschaften, insbesondere Gewebeeigenschaften, beispielsweise in einer Datenbank und/oder einer Look-Up-Tabelle, verglichen werden. Es können auch Unterscheidungskriterien, wie oben bereits dargelegt, verwendet werden, um eine klassische Auswertung umzusetzen.

In einer vorteilhaften, konkreten Ausgestaltung der vorliegenden Erfindung kann vorgesehen sein, dass wenigstens eine der wenigstens einen Messposition an einem distalen Ende des Instruments, insbesondere an einer Instrumentenspitze, vorgesehen ist. An dem distalen Ende, insbesondere der Instrumentenspitze, sind meist auch Behandlungs- und/oder Untersuchungsmittel vorgesehen, sodass die Anordnung wenigstens einer der wenigstens einen Messposition an dem distalen Ende es mit besonderem Vorteil erlaubt, in Echtzeit zu überprüfen, ob das zu behandelnde und/oder zu untersuchende Zielmaterial, insbesondere Zielgewebe, also die entsprechende Materialklasse/Gewebeklasse, erreicht ist. So wird eine erhöhte Zuverlässigkeit des Eingriffs ermöglicht. Bei einer Tumorablation kann beispielsweise vor Beginn der Behandlung überprüft werden, ob sich das distale Ende des Instruments und somit das Behandlungsmittel innerhalb von Tumorgewebe befindet. Mittels einer am distalen Ende angeordneten Zielposition kann also besonders leicht beurteilt werden, ob der Zielbereich erreicht ist.

Zweckmäßige Ausführungsbeispiele können jedoch auch vorsehen, dass mehrere Messpositionen vorgesehen sind. Während es auf diese Weise vereinfacht möglich ist, bei mehreren Behandlungs- und/oder Untersuchungsmitteln für jedes dieser Mittel das aktuell umgebende Material festzustellen oder aus anderen Gründen für verschiedene Abschnitte des medizinischen Instruments umgebende Materialklassen festzustellen, ist doch ein besonders vorteilhafter Einsatz mehrerer Messpositionen auch im Hinblick auf die Ermittlung der Positionsinformation, insbesondere auch hinsichtlich der Vorschubstrecke, denkbar.

In diesem Zusammenhang ist es besonders bevorzugt, wenn wenigstens ein Teil der mehreren Messpositionen beabstandet, insbesondere äquidistant, in Längsrichtung entlang eines in das Untersuchungsobjekt einführbaren distalen Anteils des medizinischen Instruments vorgesehen sind. Auf diese Weise werden bei zunehmendem Einschieben des distalen Anteils des medizinischen Instruments in das Untersuchungsobjekt aufeinanderfolgend immer mehr Messpositionen von Materialien des Untersuchungsobjekts, insbesondere Gewebe, bedeckt, sodass im Gegensatz zu außerhalb des Untersuchungsobjekts befindlichen Messpositionen dort Reflektion auftritt und die Intensität steigt. Dabei ist es zwar grundsätzlich denkbar, zu jeder Messposition eigene Lichtleiter für das Sendelicht und das Empfangslicht zu führen, jedoch ist es bevorzugt, wenn ein gemeinsamer des wenigstens einen Lichtleiters zur Führung des Empfangslichts von mehreren Messpositionen vorgesehen ist.

Dann kann die Auswertungseinheit zweckmäßigerweise ausgebildet sein,
- die Ermittlung der Positionsinformation in Abhängigkeit von mit steigender Anzahl innerhalb des Untersuchungsobjekts befindlichen Messpositionen steigender Intensität des Empfangslichts durchzuführen, und/oder
- für wenigstens eine Messposition bei Empfangslicht von mehreren Messpositionen beschreibenden Gesamt-Messdaten Messpositions-Messdaten durch Subtraktion von, insbesondere bei einem geringeren Vorschub isoliert gemessenen und/oder durch Subtraktion bei geringerem Vorschub hergeleiteten, Subtraktions-Messdaten von den Gesamt-Messdaten zu ermitteln.

Wird ein Instrument mit mehreren entlang seiner Längsrichtung angeordneten, der Auswertungseinheit bekannten Messpositionen in das Untersuchungsobjekt eingeführt, kann der Vorschub des medizinischen Instruments gemessen werden, indem die Lichtintensität des Gesamt-Empfangslichts aller dieser Messpositionen ausgewertet wird. Im Allgemeinen kann gesagt werden, dass ein größerer Vorschub des Instruments in das Untersuchungsobjekt aufgrund der größeren Anzahl von Messpositionen, die reflektiertes Empfangslicht erhalten, in einer höheren Intensität des Empfangslichts resultiert. Beispielsweise erhöht sich, je mehr Messpositionen von Gewebe bedeckt sind, die Rückreflektion und somit die Intensität des Empfangslichts. Immer wenn eine neue Messposition in das Untersuchungsobjekt eingeschoben wird, tritt eine Erhöhung in der Intensität des Empfangslichts als Funktion der Reflektionseigenschaften des Materials des Untersuchungsobjekts auf einen höheren Wert auf, die mit der Vorschubstrecke des medizinischen Instruments in das Untersuchungsobjekt als Positionsinformation korreliert werden kann. So kann die Position des medizinischen Instruments im Untersuchungsobjekt abgeschätzt werden.

Hierbei ist die Auswertung des Verlaufs über den Vorschub zweckmäßig, wobei, bildlich gesprochen, Stufen im Verlauf, die der Zahl bedeckter Messpositionen, die rückreflektiertes Empfangslicht erhalten, entsprechen, abgezählt werden können. Nachdem bekannt ist, wo die Messpositionen liegen, ergibt sich ein Vorschubstreckenbereich. Insbesondere existiert ein Basissignal, welches auch dann vorliegt, wenn alle Messpositionen außerhalb des Untersuchungsobjekts befindlich sind. Dieses erhöht sich stufenweise auf neue, höhere Mittelwerte der Intensität, wenn Messpositionen überdeckt werden. Alternativ oder zusätzlich zu einem "abzählen" ist es jedoch auch denkbar, durch statistische Auswertung und/oder theoretische Ermittlung Schwellwerte und/oder Schwellwertbereiche zu ermitteln, die jeweils anzeigen, wie viele Messpositionen bei einer gemessenen Intensität des Empfangslichts innerhalb des Untersuchungsobjekts befindlich sind.

Die Tatsache, dass die verschiedenen Messpositionen sukzessive in das Untersuchungsobjekt eingeführt werden, mithin durch jede vorlaufende Messposition letztlich bereits das vermessen wird, was die nachlaufende Messposition noch messen wird, lässt sich durch entsprechende Kombinatorik bei berücksichtigtem Verlauf das Empfangsspektrum auch bei Überlappung, also Messen von Empfangslicht von mehreren Messpositionen, für einzelne Messpositionen bestimmen. Befinden sich beispielsweise zwei Messpositionen innerhalb des Untersuchungsobjekts, kann man ein bereits zu einem geringeren Vorschub an der vorderen Messposition gemessenes Empfangsspektrum von dem Gesamt-Empfangsspektrum subtrahieren, um das aktuelle Messpositions-Empfangsspektrum für die vordere Messposition zu erhalten und dergleichen. Dies ermöglicht es, auch bei mehreren Messpositionen, die einen gemeinsamen Lichtleiter für das Empfangslicht nutzen, zu ermitteln, welche Materialklassen vorliegen, wie dies oben beschrieben wurde.

Dabei sei an dieser Stelle noch angemerkt, dass es bei der Verwendung einer trainierten Funktion zur Klassifizierung von Material auch denkbar ist, diese bei Überlappung von Empfangslicht mehrerer Messpositionen so zu trainieren, dass auch eine Trennung und eine Bestimmung der entsprechenden Materialklassen erlaubt wird, wobei dann eine Zuordnung zu Messpositionen beispielsweise aufgrund von Hintergrundwissen, insbesondere von Bilddaten, stattfinden kann.

Insbesondere ist es allgemein bei Vorliegen von Bilddaten des Untersuchungsobjekts, welche insbesondere entsprechende Materialabschnitte, insbesondere Gewebeabschnitte, zeigen, möglich, Gesamt-Empfangsspektren mit denkbaren Kombinationen so abzugleichen, so dass eine genauere Ortsbestimmung des medizinischen Instruments im Untersuchungsobjekt möglich wird als bei Verwendung nur einer einzigen Messposition, beispielsweise an der Spitze des Instruments. Diese Verbesserung tritt nicht nur bei der Kombination mit Bilddaten auf, sondern auch bereits unter Berücksichtigung bereits von vorlaufenden Messpositionen durchlaufener Materialklassen/Materialabschnitte. Generell sei noch angemerkt, dass selbstverständlich bei der Auswertung das Wissen über die Anordnung der Messpositionen an dem medizinischen Instrument berücksichtigt wird.

Es kann vorgesehen sein, dass der Lichtleiter wenigstens eine optische Faser umfasst. Beispielsweise kann eine sogenannte "double-clad fiber" verwendet werden, die üblicherweise drei optische Schichten aufweist, welche unterschiedliche Brechungsindices aufweisen und bei denen der Kern und die erste darum gelegte Schicht beide zum Lichttransport verwendet werden können, hier beispielsweise zum Transport des Sendelichts und des Empfangslichts über unterschiedliche "Kanäle" der doppelt verkleideten Faser. Selbstverständlich können auch andere Fasern und Faserstrukturen als Lichtleiter verwendet werden, beispielsweise eine Multimodenfaser.

Grundsätzlich ist es denkbar, dass für wenigstens eine der wenigstens einen Messposition unterschiedliche Lichtleiter für die Führung des Sendelichts und des Empfangslichts vorgesehen sind. Möglich und bevorzugt ist es aber auch, dass das Messgerät, wenn für wenigstens eine der wenigstens einen Messposition einer des wenigstens einen Lichtleiters zur Führung sowohl des Sendelichts als auch des Empfangslichts vorgesehen ist, eine optische Trenneinheit zur Aufteilung des Empfangslicht und des Sendelichts aufweist. Hierbei können beispielsweise übliche Strahlteiler eingesetzt werden.

Der Lichtdetektor kann im Rahmen der vorliegenden Erfindung beispielsweise einen Photon-Multiplier und/oder einen Photonenzähler umfassen. Auch andere, im Stand der Technik grundsätzlich bekannte Ausgestaltungen sind denkbar.

Der wenigstens eine des wenigstens einen Lichtleiters zur Führung von Sendelicht an wenigstens eine der wenigstens einen Messposition kann an dieser Messposition eine Störstelle zur Auskopplung des Sendelichts aufweisen. Dies ist insbesondere dann zweckmäßig, wenn ein Lichtleiter für mehrere Messpositionen, beispielsweise entlang der Länge des medizinischen Instruments, verwendet wird, da dann, beispielsweise äquidistant, Störstellen an den entsprechenden Messpositionen vorgesehen werden, um jeweils Sendelicht auszukoppeln.

Konkret kann der Lichtleiter, wie bereits erwähnt, innerhalb des Instruments integriert geführt werden, beispielsweise in einer Nut eines Instrumentenschafts. Möglich ist es aber auch, den Lichtleiter vollständig innerhalb des Instruments zu führen und an den Messpositionen lichtdurchlässige Fenster vorzusehen.

Wie bereits erwähnt, kann vorgesehen sein, dass das Instrument, insbesondere an seiner Instrumentenspitze, ein Behandlungsmittel zur Behandlung einer Zielgewebeklasse, insbesondere von Tumorgewebe, aufweist, insbesondere das Instrument eine Ablationsnadel ist. Insbesondere im Hinblick auf die zerstörende Behandlung von insbesondere krankhaftem Zielgewebe ist die vorliegende Erfindung besonders zweckmäßig, da überprüft werden kann, ob der Zielbereich auch tatsächlich erreicht worden ist.

Die Eingriffsanordnung kann vorzugsweise ferner eine Bildgebungseinrichtung mit einer Patientenliege für das Untersuchungsobjekt und, insbesondere als Teil der Steuereinrichtung, eine Ansteuereinheit aufweisen, die zur Ansteuerung der Patientenliege und/oder einer Aufnahmeanordnung der Bildgebungseinrichtung zur Positionierung für eine Aufnahme von Bilddaten mit der Bildgebungseinrichtung unter Verwendung der Positionsinformation ausgebildet ist. Das bedeutet, wenn eine Verbindung mit der Bildgebungseinrichtung und deren Positionierungsmitteln vorliegt, kann die Positionsinformation, insbesondere hinsichtlich der Vorschubstrecke des medizinischen Instruments, auch genutzt werden, um die Bildgebungseinrichtung korrekt auf eine folgende Bildaufnahme einzustellen, insbesondere so, dass die Instrumentenspitze erfasst wird.

Konkret ist es hierbei zweckmäßig, die Positionsinformation zu nutzen, um den Patiententisch und/oder eine Aufnahmeanordnung der Bildgebungseinrichtung automatisch in eine Zielposition für einen Kontrollscan zu verbringen. Dies ist insbesondere bei Computertomografie-Einrichtungen als Bildgebungseinrichtungen zweckmäßig, wenn der Eingriff nicht in einer einzigen Computertomografie-Schicht stattfindet, sondern hier mit zunehmendem Vorschub ein Wechsel der Computertomographie-Schicht zweckmäßig ist. So ist auch ein weiterer, zweckmäßiger, den Eingriff erleichternder Nutzen der Positionsinformation gegeben.

Neben der Eingriffsanordnung betrifft die Erfindung auch ein computerimplementiertes Verfahren zur Ermittlung einer Positionsinformation eines in einem Eingriffsgebiet eines Untersuchungsobjekt befindlichen länglichen medizinischen Instruments, wobei innerhalb des medizinischen Instruments wenigstens ein Lichtleiter zu wenigstens einer distalen Messposition, in der der Lichtleiter zum Senden und Empfangen von Licht offenliegt, geführt ist, und ein extern zum Untersuchungsobjekt angeordnetes Messgerät, das mit dem proximalen Ende des wenigstens einen Lichtleiters verbunden ist, verwendet wird, aufweisend die Schritte:
- mittels einer Lichtquelle des Messgeräts, Erzeugung von durch wenigstens einen des wenigstens einen Lichtleiters zu der wenigstens einen Messposition zu führendem Sendelicht eines Sendespektrums,
- mittels eines Lichtdetektors des Messgeräts, Empfang von Empfangslicht, das von der wenigstens einen Messposition über wenigstens einen des wenigstens einen Lichtleiters zu dem Messgerät geführt ist,
- mittels einer Auswertungseinheit des Messgeräts, Auswertung von das Empfangsspektrum des Empfangslichts beschreibenden Messdaten in Kenntnis des Sendespektrums zur Ermittlung einer die Position des medizinischen Instruments in dem Untersuchungsobjekt beschreibenden Positionsinformation auf der Grundlage von Reflektionseigenschaften von Materialien, insbesondere Geweben, des Untersuchungsobjekts, und
- Ausgabe der Positionsinformation an einer Darstellungseinrichtung.

Sämtliche Ausführungen bezüglich der erfindungsgemäßen Eingriffsanordnung lassen sich analog auf das erfindungsgemäße Verfahren übertragen und umgekehrt, sodass mit diesem ebenso die bereits genannten Vorteile erhalten werden können.

Denkbar ist ferner auch ein Computerprogramm, welches Programmmittel aufweist, welche bei Ausführung des Computerprogramms auf einer Steuereinrichtung einer Eingriffsanordnung, diese veranlassen, ein erfindungsgemäßes Verfahren auszuführen. Das Computerprogramm kann auf einem elektronisch lesbaren Datenträger gespeichert sein.

Weitere Vorteile und Einzelheiten der vorliegenden Erfindung ergeben sich aus den im Folgenden beschriebenen Ausführungsbeispielen sowie anhand der Zeichnungen. Dabei zeigen:
- Fig. 1: eine allgemeine funktionale Prinzipskizze einer erfindungsgemäßen Eingriffsanordnung,
- Fig. 2: eine Interaktion von Licht mit Material erläuternde Darstellung,
- Fig. 3: den funktionalen Aufbau eines Messgeräts in einer ersten Ausführungsvariante,
- Fig. 4: den funktionalen Aufbau des Messgeräts in einer zweiten Ausführungsvariante,
- Fig. 5: eine Prinzipskizze eines medizinischen Instruments in einem ersten Ausführungsbeispiel der Eingriffsanordnung,
- Fig. 6: eine beispielhafte Struktur eines Untersuchungsobjekts,
- Fig. 7: rein schematisch einen sich ergebenden Messdatenverlauf bei Vorschub durch die Struktur der Fig. 6,
- Fig. 8: eine Prinzipskizze eines medizinischen Instruments in einem zweiten Ausführungsbeispiel der Eingriffsanordnung,
- Fig. 9: ein Detail eines Lichtleiters im Bereich einer Messposition,
- Fig. 10: einen Verlauf der Intensität von Empfangslicht mit dem Vorschub bei mehreren Messpositionen, und
- Fig. 11: einen Ablaufplan eines Ausführungsbeispiels des erfindungsgemäßen Verfahrens.

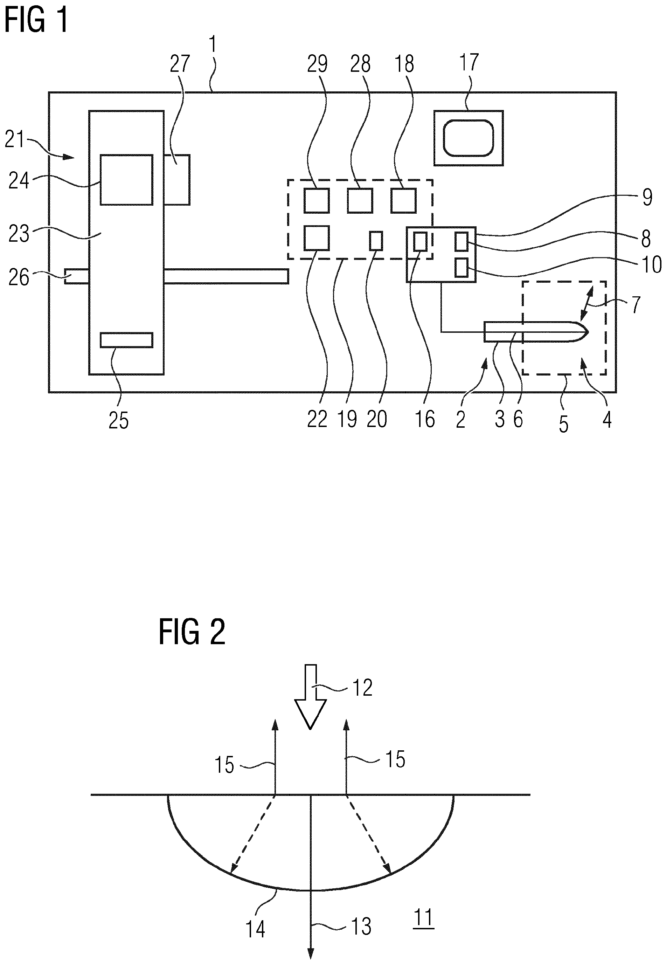

Fig. 1 zeigt eine funktionale Prinzipskizze einer erfindungsgemäßen Eingriffsanordnung 1 zur Durchführung eines minimalinvasiven Eingriffs. Die Eingriffsanordnung 1 umfasst vorliegend als medizinisches Instrument 2 eine längliche, zumindest im wesentlichen starre Nadel 3, beispielsweise eine Ablationsnadel zur Behandlung von Tumoren, beispielsweise durch Hitze, Kälte, Hochfrequenzstrahlung oder dergleichen. Hierzu weist die Nadel 3 auch ein in Fig. 1 der Übersichtlichkeit halber nicht näher gezeigtes Behandlungsmittel auf.

Das Instrument 2 kann mit einem distalen Anteil, umfassend die Spitze am distalen Ende 4 in ein (nicht zur Eingriffsanordnung 1 gehöriges und daher nur angedeutetes) Untersuchungsobjekt 5 eingebracht werden. Innerhalb des medizinischen Instruments 2 verläuft wenigstens ein Lichtleiter 6 zu wenigstens einer distalen, mithin am distalen Anteil vorgesehenen, Messposition, wo Sendelicht aus dem Lichtleiter 6 austreten kann und gegebenenfalls von an der Messposition vorliegendem Material, insbesondere des Untersuchungsobjekts 5, reflektiertes Empfangslicht wieder in den Lichtleiter 6 eintreten kann, vergleiche den Doppelpfeil 7.

Das Sendelicht wird mittels einer Lichtquelle 8, beispielsweise einem Laser, in einem Messgerät 9, mit dem das proximale Ende des Lichtleiters 6 ersichtlich verbunden ist, erzeugt. Das Messgerät umfasst ferner auch einen Lichtdetektor 10, mit dem das Empfangslicht detektiert werden kann. Der Lichtdetektor 10 kann beispielsweise einen Photomultiplier und/oder einen Photonenzähler umfassen.

Fig. 2 zeigt schematisch die Interaktion von Licht mit einem Material 11, beispielsweise einem Gewebe. Der Pfeil 12 symbolisiert einfallendes Sendelicht. Dieses Sendelicht wird nun zum Teil transmittiert (Transmission, Pfeil 13), zum Teil absorbiert (Absorption, symbolisiert durch den Bogen 14) und zum Teil reflektiert (Reflektion, vergleiche Pfeile 15). Die Reflexion umfasst vorliegend sowohl die gerichtete Reflektion als auch die diffuse Reflektion. Der Anteil an reflektiertem Sendelicht und somit das Empfangslicht wird beschrieben durch die Reflektionseigenschaften des Materials 11, beispielsweise den Reflektionskoeffizienten.

Ist das Sendespektrum des Sendelichts bekannt, welches vorliegend zweckmäßigerweise mehrere Wellenlängen und/oder Wellenlängenbereiche umfasst (multispektrale Messung) und im optischen und/oder Nahinfrarot-Bereich liegt, kann zwischen unterschiedlichen Materialien 11 bzw. unterschiedlichen Materialklassen durch Betrachtung der von dem Lichtdetektor 10 aufgenommenen Messdaten, die das Empfangsspektrum, insbesondere also Intensitäten bei verschiedenen Wellenlängen, beschreiben, unterschieden werden. In einem einfachen Ansatz, worauf im Folgenden ebenso noch näher eingegangen werden wird, kann auch lediglich durch Zunahme der Intensität des Empfangslichts geschlossen werden, dass nun eine Reflexion durch Material 11 vorliegt, die zuvor nicht vorhanden war.

Zurückkehrend zu Fig. 1 wertet eine Auswertungseinheit 16 des Messgeräts 9 die Messdaten unter Kenntnis der Reflektionseigenschaften und des Sendespektrums aus, um eine Positionsinformation zur Position des medizinischen Instruments 2 im Untersuchungsobjekt 5 zu ermitteln. Allgemein gesprochen kann die Positionsinformation beispielsweise eine Materialklasse, insbesondere Gewebeklasse, innerhalb der sich wenigstens eine der wenigstens einen Messposition befindet, genauso beschreiben wie eine Vorschubstrecke oder einen Vorschubstreckenbereich (als grobe Positionsangabe) und/oder einen, beispielsweise aus Bilddaten ersichtlichen, Materialabschnitt, insbesondere Gewebeabschnitt, in dem sich wenigstens eine der wenigstens einen Messposition bzw. ein bestimmter Anteil des medizinischen Instruments 2 befindet. Konkrete Beispiele werden im Folgenden noch dargelegt.

Die Auswertungseinheit 16 kann eine trainierte Funktion, alternativ oder zusätzlich jedoch auch Unterscheidungskriterien, die auf Basis bekannter Reflexionsverhalten erwarteter Materialien bestimmter Materialklassen definiert sind, und/oder eine Datenbank oder Look-Up-Tabelle, in der Messdaten bzw. Empfangsspektren Materialklassen zugeordnet sind, nutzen, um eine Materialklasse für wenigstens eine der wenigstens einen Messposition herzuleiten. Wird eine trainierte Funktion verwendet, kann diese ein neuronales Netz, insbesondere ein tiefes neuronales Netz, umfassen und aufgrund von bei bekannten Materialien der Materialklassen aufgenommenen Trainings-Messdaten trainiert sein. Die Ermittlung der Materialklasse, also die Klassifizierung, kann auf Basis der Messdaten für Empfangslicht einer bestimmten Messposition (Messpositions-Messdaten) erfolgen, aber auch auf Basis von überlagertes Empfangslicht mehrerer Messpositionen beschreibenden Gesamt-Messdaten, wobei es auch möglich ist, bei Betrachtung des Verlaufs der Messdaten über die Vorschubstrecke in das Untersuchungsobjekt 5 durch Kombinatorik aus Gesamt-Messdaten, insbesondere durch Subtraktion von Subtraktions-Messdaten, Messpositions-Messdaten für einzelne Messpositionen herzuleiten. Auch allgemein kann es für die Auswertungseinheit 16 zweckmäßig sein, den Verlauf der Messdaten über die Vorschubstrecke bei der Ermittlung der Positionsinformation zu verwenden.

Zweckmäßig ist es ferner, wenn Hintergrundwissen, insbesondere in Form von Bilddaten, bei der Ermittlung der Positionsinformation durch die Auswertungseinheit 16 herangezogen wird. Bilddaten des Untersuchungsobjekts 5, beispielsweise Planungs-Bilddaten und/oder auch Bilddaten eines Kontrollscans, können bereits Materialabschnitte, insbesondere Gewebeabschnitte, anzeigen, die durch das medizinische Instrument 2 bei Vorschub in das Untersuchungsobjekt 5 zwangsläufig durchquert werden müssen und denen anhand der Messdaten Messpositionen zugeordnet werden können. Nachdem vorliegend die Positionsinformation auch an einer Darstellungseinrichtung 17, hier einem Monitor, der Eingriffsanordnung 1 ausgegeben werden, um eine den Eingriff durchführende Person zu unterstützen, kann es zweckmäßig sein, eine Darstellung zu erzeugen, die die Positionsinformation, beispielsweise überlagert, zu den Bilddaten anzeigt.

Die Auswertungseinheit 16 sowie eine die Darstellungseinrichtung 17 zur Ausgabe der Positionsinformation ansteuernde Ausgabeeinheit 18 können auch Teil einer Steuereinrichtung 19 der Eingriffsanordnung 1 sein, welche vorliegend teilweise auch extern zu dem Messgerät 9 angeordnet ist. Die Steuereinrichtung 19 umfasst wenigstens einen Prozessor und wenigstens ein Speichermittel 20.

Im vorliegenden Ausführungsbeispiel umfasst die Eingriffsanordnung 1 auch eine Bildgebungseinrichtung 21, die zur Aufnahme wenigstens eines Teils der bereits genannten Bilddaten durch eine Aufnahmeeinheit 22 der Steuereinrichtung 19 betreibbar ist. Bei der Bildgebungseinrichtung 21 handelt es sich vorliegend um eine Computertomografie-Einrichtung, die eine Gantry 23 mit einer darin geführten Aufnahmeanordnung, umfassend einen Röntgenstrahler 24 und einen Röntgendetektor 25, sowie eine Patientenliege 26 zur Platzierung des Untersuchungsobjekts aufweist. Es sind auch andere Bildgebungseinrichtungen denkbar, beispielsweise C-Bogen-Röntgeneinrichtungen.

An der Gantry 23 ist vorliegend auch eine Lichtführungseinrichtung 27 der Eingriffsanordnung 1 angeordnet, vorliegend eine Laserführungseinrichtung. Nachdem die Steuereinrichtung 19 vorliegend auch eine Planungseinheit 28 aufweist, kann mittels dieser basierend auf einem Planungs-Bilddatensatz, der mit der Bildgebungseinrichtung 21 aufgenommen wird, eine Planungsinformation ermittelt werden, die vorliegend einen geplanten Instrumentenpfad in einen zu behandelnden Zielbereich, hier ein Tumor, mit zu behandelndem Zielgewebe, hier Tumorgewebe, derart beschreibt, dass ein Soll-Eintrittspunkt des medizinischen Instruments 2, eine Soll-Orientierung des medizinischen Instruments 2 sowie eine gewünschte Vorschubstrecke bzw. die Lage des Zielbereichs an sich darin enthalten oder daraus herleitbar sind. Dies erlaubt es einer Ansteuereinheit 29 der Steuereinrichtung 19, die Lichtführungseinrichtung 27 derart anzusteuern, dass auf der Oberfläche des Untersuchungsobjekts 5 der Soll-Eintrittspunkt markiert werden kann und, beispielsweise mittels eines Laserkreuzes, die Orientierung gemäß der Planungsinformation vorgegeben werden kann. Zu dieser Unterstützung hinsichtlich der Orientierung und des Eintrittspunkt kommt nun dank des Messgeräts 9 und des Lichtleiters 6 und der dadurch ermöglichten Ermittlung und Ausgabe der Positionsinformation eine weitere, hervorragende Unterstützung der den Eingriff durchführenden Person, die nun beispielsweise optimal beurteilen kann, ob der Zielbereich wie gewünscht erreicht ist bzw. das medizinische Instrument zur Durchführung der Behandlung geeignet positioniert ist.

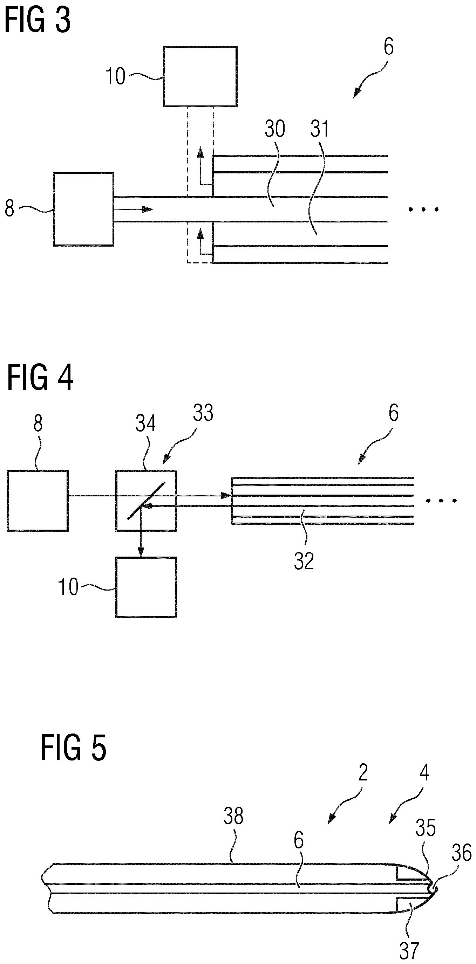

Für den Lichtleiter 6 sowie das Einkoppeln und Auskoppeln von Sendelicht bzw. Empfangslicht im Messgerät 9 existieren im Rahmen der vorliegenden Erfindung verschiedene Möglichkeiten, von denen zwei beispielhaft in den Figuren 3 und 4 erläutert sind. Fig. 3 zeigt einen Lichtleiter 6, der als doppelt ummantelte optische Faser (double-clad fiber) ausgebildet ist. Dabei wird vorliegend der Kern 30 zum Transport des Sendelichts ausgehend von der Lichtquelle 8 genutzt, während die innere Ummantelung 31 für den Rücktransport des Empfangslichts eingesetzt wird, welches dann, wie angezeigt, dem Lichtdetektor 10 zugeführt wird. Statt einer doppelt ummantelten optischen Faser kann auch eine multimodale Faser eingesetzt werden; ferner ist es denkbar, zwei getrennte Fasern bzw. Lichtleiter 6 für das Sendelicht und das Empfangslicht zu verwenden.

In der Ausführungsform der Fig. 4 ist als Lichtleiter 6 eine übliche, auch einfach ummantelte optische Faser gezeigt, deren Kern 32 sowohl zum Transport des Sendelichts von der Lichtquelle 8 als auch zum Transport des Empfangslichts zu dem Lichtdetektor 10 verwendet wird, wobei als optische Trenneinheit 33 zur Aufteilung des Sendelichts und Empfangslichts vorliegend ein 50:50-Strahlteiler 34 (beam splitter) verwendet wird.

Fig. 5 zeigt nun ein erstes konkretes Ausführungsbeispiel eines medizinischen Instruments 2, wobei vorliegend der Einfachheit halber nur der distale Anteil mit dem distalen Ende 4, also der Instrumentenspitze 35, gezeigt ist. Der in diesem Fall genau eine Lichtleiter 6 ist zu einer Messposition 36 an der Instrumentenspitze 35 geführt. An der Instrumentenspitze 35 ist vorliegend auch ein Behandlungsmittel 37, beispielsweise ein Ablationsmittel, vorgesehen. Der Lichtleiter 6 ist in diesem Beispiel innerhalb des Instrumentenschafts 38 geführt. Die Messposition 36 kann beispielsweise durch ein das Ende des Lichtleiters 6 abdeckendes Fenster, aber auch durch das Ende des Lichtleiters 6 direkt gebildet werden.

In einer derartigen Ausgestaltung ist es besonders zweckmäßig, wenn durch die Auswertungseinheit die Materialklasse an der Messposition 36 aus den Messdaten, wie oben beschrieben, bestimmt wird, da dann insbesondere überprüft werden kann, ob das Zielgewebe, also der Zielbereich, erreicht ist. Dies kann nicht nur anhand einer Klassifikation durch die trainierte Funktion, die Entscheidungskriterien und/oder die Datenbank/Look-Up-Tabelle erfolgen, sondern zusätzlich oder alternativ auch durch Hinzunahme der in diesem Fall zweckmäßig annotierten Bilddaten, da beispielsweise über den Verlauf der Messdaten Übergänge zwischen unterschiedlichen Materialklassen, hier Gewebeklassen, gezählt werden können. Bevorzugt ist jedoch die Klassifizierung, welche insbesondere durch das Abzählen von Materialübergängen plausibilisiert werden kann.

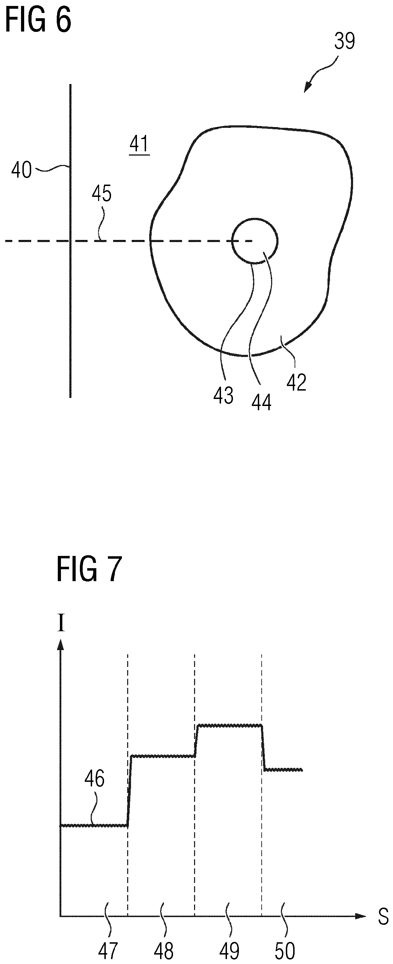

Fig. 6 zeigt schematisch ein Eingriffsgebiet 39 des Untersuchungsobjekts 5 mit der Haut 40 als äußere Oberfläche, darauf nach innen folgendem Fettgewebe 41, dem Übergang zu Organ-Parenchym 42 sowie einem Tumor 43, der den Zielbereich bildet, sodass das Tumorgewebe 44 entsprechend das Zielgewebe darstellt. Ein geplanter Instrumentenpfad 45 ist ebenso gezeigt. Bei dem Organ kann es sich beispielsweise um eine Leber handeln.

Fig. 7 zeigt rein schematisch den Verlauf 46 einer aus den Messdaten herleitbaren, die Unterscheidung zwischen den Gewebeklassen Fettgewebe, Organ-Parenchym und Tumorgewebe erlaubenden Größe I über der Vorschubstrecke s. In einem Bereich 47 befindet sich die Messposition 36 noch außerhalb des Untersuchungsobjekt 5, hier Patienten, weswegen nur ein Basis-Signal vorliegt. Im Bereich 48 wird das medizinische Instrument 2, konkret die Messposition 36, durch das Fettgewebe 41 geführt, welches das Sendelicht gemäß den Reflektionseigenschaften von Fettgewebe 41 teilweise in Empfangslicht reflektiert und somit für einen anderen Wert der Größe sorgt. Sodann erfolgt der Übergang in einen Bereich 49 (Organ-Parenchym 42), bevor im Bereich 50 nach einem weiteren Übergang auf einen wiederum neuen Wert der Größe das Zielgewebe (Tumorgewebe 44) erreicht wird.

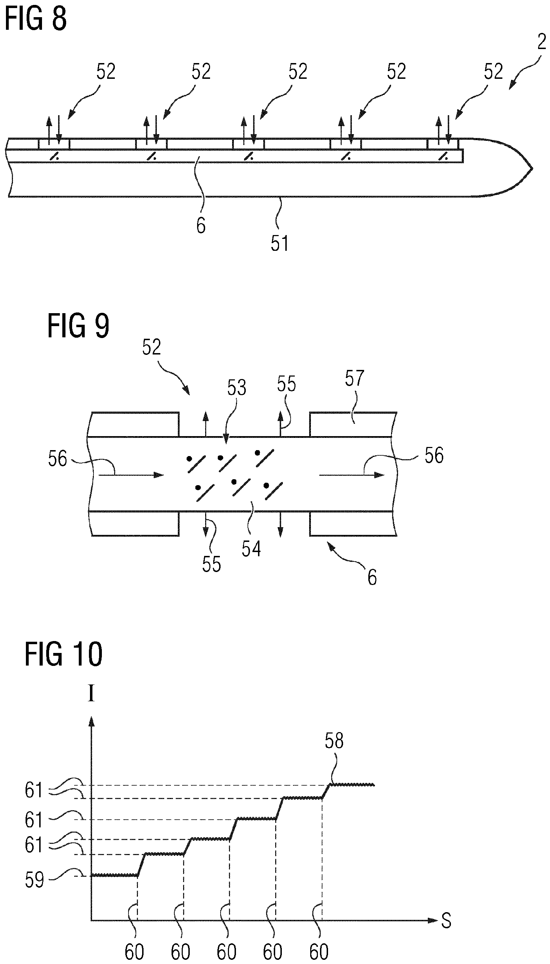

Fig. 8 erläutert ein zweites konkretes Ausführungsbeispiel eines medizinischen Instruments 2, welches vorliegend einen in einer Nut des Instrumentenschafts 51 geführten Lichtleiter 6 aufweist, welcher mehreren, äquidistant entlang der Länge des Instruments 2 vorgesehenen Messpositionen 52 zugeordnet ist. Das bedeutet, der Lichtleiter 6 führt sowohl das Sendelicht als auch das Empfangslicht für alle diese Messpositionen 52, wobei vorliegend beispielhaft fünf Messpositionen 52 verwendet werden, allgemein jedoch auch eine andere Anzahl, beispielsweise vier bis zehn, verwendet werden kann. Wie in Fig. 9 genauer gezeigt ist, sind an jeder Messposition 52 Störstellen 53 im Lichtleiter 6, konkret im Kern 54 der optischen Faser, vorgesehen, die durch Pfeile 55 gekennzeichnete Anteile des durch Pfeile 56 gekennzeichneten Sendelichts auskoppeln. Die Ummantelung 57 der optischen Faser kann an diesen Stellen unterbrochen sein.

Auch bei diesem zweiten Ausführungsbeispiel kann der Lichtleiter 6 an den Messpositionen 52 entsprechend freiliegen, möglich ist es jedoch auch, Fenster zu verwenden.

In diesem zweiten Ausführungsbeispiel wird ausgenutzt, dass jede in das Untersuchungsobjekt 5 eintretende Messposition 52 die Intensität des Gesamt-Empfangslichts erhöht, da ab diesem Zeitpunkt Reflektion von Material, insbesondere Gewebe, auftritt. Dies ist durch den charakteristischen Intensitätsverlauf 58, wie er in Fig. 10 rein schematisch dargestellt ist, verdeutlicht. Wiederum wird dabei von einem Basis-Signal 59, bei dem alle Messpositionen 52 außerhalb des Untersuchungsobjekts 5 angeordnet sind, ausgegangen, wobei bei jedem Vorschubspunkt 60 eine neue Messposition 52 von Material überdeckt wird, sodass die Intensität auf einen neuen Mittelwert 61 ansteigt. Nach fünf derartigen Stufen sind alle Messpositionen 52 im Untersuchungsobjekt 5 befindlich. Durch Zählen der Stufen im Verlauf 58 kann also bereits erkannt werden, wie viele Messpositionen 52 sich im Untersuchungsobjekt 5 befinden, woraus als Positionsinformation bereits ein Vorschubstreckenbereich angegeben werden kann.

Besonders bevorzugt werden jedoch auch hier Materialklassen bestimmt, nachdem sich selbstverständlich auch in dem Verlauf 58 zugrunde liegenden Messdaten charakteristische Reflektionseigenschaften sowie Materialübergänge erkennen lassen. Hierzu können, wie dargelegt, Messposition-Messdaten für einzelne Messpositionen 52 extrahiert werden oder aber auch eine Gesamtauswertung der Gesamt-Messdaten erfolgen. Auch hier ist eine Nutzung von Bilddaten bzw. eine Verknüpfung mit diesen zweckmäßig.

Es sei angemerkt, dass die Ausführungsbeispiele der Figuren 5 bis 7 und 8 bis 10 selbstverständlich auch kombiniert werden können, beispielsweise zu den Messpositionen 52 und den diese verbindenden Lichtleiter 6 auch die weitere Messposition 36 an der Spitze 35 sowie der Lichtleiter 6 hinzugefügt werden können.

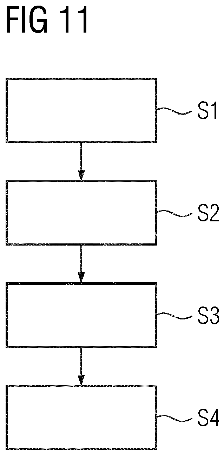

Fig. 11 zeigt schließlich noch einen allgemeinen Ablaufplan eines Ausführungsbeispiels des erfindungsgemäßen Verfahrens, genauer dessen wesentliche Schritte. Dabei lassen sich die Ausführungen zu den bisherigen Ausführungsbeispielen selbstverständlich analog entsprechend übertragen, beispielsweise was weitere Schritte und/oder die Konkretisierung von Schritten angeht.

In einem Schritt S1 wird mittels der Lichtquelle 8 Sendelicht bereitgestellt und über den Lichtleiter 6 zu wenigstens einer der wenigstens einen Messposition 36, 52 geführt. Abhängig von den Reflektionseigenschaften umgebenden Materials entsteht nun an die Messposition 36, 52 zurückreflektiertes Empfangslicht, dass durch den oder einen weiteren Lichtleiter 6 zu dem Lichtdetektor 10 geführt wird, der in einem Schritt S2 die Messdaten, die das Empfangsspektrum des Empfangslichts beschreiben, aufnimmt.

In einem Schritt S3 werden die Messdaten dann in der Auswertungseinheit 16 zu der Positionsinformation ausgewertet. Die so erhaltene Positionsinformation kann in einem Schritt S4 an der Darstellungseinrichtung 17 ausgegeben werden, insbesondere gesteuert durch die Ausgabeeinheit 18 und/oder in einer auf den Bilddaten basierenden Darstellung.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

Unabhängig vom grammatikalischen Geschlecht eines bestimmten Begriffes sind Personen mit männlicher, weiblicher oder anderer Geschlechteridentität mit umfasst.

## Patentansprüche

1. Medizinische Eingriffsanordnung (1), umfassend
- ein längliches medizinisches Instrument (2) zum teilweisen Einführen in ein Eingriffsgebiet (39) eines Untersuchungsobjekts (5), wobei innerhalb des medizinischen Instruments (2) wenigstens ein Lichtleiter (6) zu wenigstens einer distalen Messposition (36, 52), in der der Lichtleiter (6) zum Senden und Empfangen von Licht offenliegt, geführt ist, und
- ein extern zum Untersuchungsobjekt (5) angeordnetes Messgerät (9), das mit dem proximalen Ende des wenigstens einen Lichtleiters (6) verbunden ist und aufweist:
- eine Lichtquelle (8) zur Erzeugung von durch wenigstens einen des wenigstens einen Lichtleiters (6) zu der wenigstens einen Messposition (36, 52) zu führendem Sendelicht eines Sendespektrums,
- einen Lichtdetektor (10) zum Empfang von Empfangslicht, das von der wenigstens einen Messposition (36, 52) über wenigstens einen des wenigstens einen Lichtleiters (6) zu dem Messgerät (9) geführt ist, und
- eine Auswertungseinheit (16) zur Auswertung von das Empfangsspektrum des Empfangslichts beschreibenden Messdaten in Kenntnis des Sendespektrums zur Ermittlung einer die Position des medizinischen Instruments (2) in dem Untersuchungsobjekt (5) beschreibenden Positionsinformation auf der Grundlage von Reflektionseigenschaften von Materialien, insbesondere Geweben, des Untersuchungsobjekts (5);
und
- eine Darstellungseinrichtung (17) zur Ausgabe der Positionsinformation.

2. Eingriffsanordnung nach Anspruch 1, **dadurch gekennzeichnet, dass** die Auswertungseinheit (16) ausgebildet ist, bei der Ermittlung der Positionsinformation einen über den Vorschub in das Untersuchungsobjekt (5) entstehenden Verlauf der Messdaten zu verwenden, und/oder das Sendespektrum mehrere Wellenlängen und/oder Wellenlängenbereiche abdeckt.

3. Eingriffsanordnung nach Anspruch 1 oder 2, **dadurch gekennzeichnet, dass** die Auswertungseinheit (16) ausgebildet ist, als Positionsinformation und/oder bei der Ermittlung der Positionsinformation aus den Messdaten wenigstens eine Materialklasse, insbesondere Gewebeklasse, des an wenigstens einer der wenigstens einen Messposition (36, 52) das Sendelicht in das Empfangslicht reflektierenden Materials und/oder einen Übergang zwischen solchen Materialklassen, insbesondere Gewebeklassen, zu ermitteln.

4. Eingriffsanordnung nach Anspruch 3, **dadurch gekennzeichnet, dass** die Positionsinformation eine Anordnung wenigstens einer der wenigstens einen Messposition (36, 52) in einem Materialabschnitt des Untersuchungsobjekts beschreibt.

5. Eingriffsanordnung nach Anspruch 2 oder 3, **dadurch gekennzeichnet, dass** die Auswertungseinheit (16) zur Berücksichtigung von Bilddaten des Eingriffsgebiets (39) des Untersuchungsobjekts (5) bei der Klassifizierung und/oder zur Zuordnung wenigstens einer der wenigstens einen Messposition (36, 52) zu einem bzw. dem in den Bilddaten sichtbaren Materialabschnitt ausgebildet ist.

6. Eingriffsanordnung nach Anspruch 5, **dadurch gekennzeichnet, dass** die Eingriffsanordnung (1) ferner eine Ausgabeeinheit (18) aufweist, welche zur Ausgabe einer auf den Bilddaten basierenden, die Positionsinformation anzeigenden Darstellung ausgebildet ist.

7. Eingriffsanordnung nach einem der Ansprüche 3 bis 6, **dadurch gekennzeichnet, dass** die Auswertungseinheit (16) zur Verwendung einer trainierten Funktion zur Klassifizierung ausgebildet ist.

8. Eingriffsanordnung nach einem der vorangehenden Ansprüche, **dadurch gekennzeichnet, dass** wenigstens eine der wenigstens einen Messposition (36) an einem distalen Ende (4) des Instruments (2), insbesondere an einer Instrumentenspitze (35), vorgesehen ist.

9. Eingriffsanordnung nach einem der vorangehenden Ansprüche, **dadurch gekennzeichnet, dass** mehrere Messpositionen (36, 52) vorgesehen sind.

10. Eingriffsanordnung nach Anspruch 9, **dadurch gekennzeichnet, dass** wenigstens ein Teil der mehreren Messpositionen (52) beabstandet, insbesondere äquidistant, in Längsrichtung entlang eines in das Untersuchungsobjekt (5) einführbaren distalen Anteils des medizinischen Instruments (2) vorgesehen sind.

11. Eingriffsanordnung nach Anspruch 10, **dadurch gekennzeichnet, dass** ein gemeinsamer des wenigstens einen Lichtleiters (6) zur Führung des Empfangslichts von mehreren Messpositionen (52) vorgesehen ist und die Auswertungseinheit (16) ausgebildet ist,
- die Ermittlung der Positionsinformation in Abhängigkeit von mit steigender Anzahl innerhalb des Untersuchungsobjekts (5) befindlichen Messpositionen (52) steigender Intensität des Empfangslichts durchzuführen, und/oder
- für wenigstens eine Messposition (52) bei Empfangslicht von mehreren Messpositionen (52) beschreibenden Gesamt-Messdaten Messpositions-Messdaten durch Subtraktion von, insbesondere bei einem geringeren Vorschub isoliert gemessenen und/oder durch Subtraktion bei geringerem Vorschub hergeleiteten, Subtraktions-Messdaten von den Gesamt-Messdaten ermittelt werden.

12. Eingriffsanordnung nach einem der vorangehenden Ansprüche, **dadurch gekennzeichnet, dass** der Lichtleiter (6) wenigstens eine optische Faser umfasst und/oder dass für wenigstens eine der wenigstens einen Messposition (36, 52) unterschiedliche Lichtleiter (6) für die Führung des Sendelichts und des Empfangslichts vorgesehen sind und/oder dass das Messgerät (9), wenn für wenigstens eine der wenigstens einen Messposition (36, 52) einer des wenigstens einen Lichtleiters (6) zur Führung sowohl des Sendelichts als auch des Empfangslichts vorgesehen ist, eine optische Trenneinheit (33) zur Aufteilung des Empfangslicht und des Sendelichts aufweist, und/oder dass der wenigstens eine des wenigstens einen Lichtleiters (6) zur Führung von Sendelicht an wenigstens eine der wenigstens einen Messposition (36, 52) an dieser Messposition (36, 52) eine Störstelle (53) zur Auskopplung des Sendelichts aufweist.

13. Eingriffsanordnung nach einem der vorangehenden Ansprüche, **dadurch gekennzeichnet, dass** das Instrument (2) an seiner Instrumentenspitze (35) ein Behandlungsmittel (37) zur Behandlung einer Zielgewebeklasse, insbesondere von Tumorgewebe, aufweist, insbesondere das Instrument (2) eine Ablationsnadel ist.

14. Eingriffsanordnung nach einem der vorangehenden Ansprüche, **dadurch gekennzeichnet, dass** sie ferner eine Bildgebungseinrichtung (21) mit einer Patientenliege (26) für das Untersuchungsobjekt (5) und eine Ansteuereinheit (29) aufweist, die zur Ansteuerung der Patientenliege (26) und/oder einer Aufnahmeanordnung der Bildgebungseinrichtung (21) zur Positionierung für eine Aufnahme von Bilddaten mit der Bildgebungseinrichtung (21) unter Verwendung der Positionsinformation ausgebildet ist.

15. Computerimplementiertes Verfahren zur Ermittlung einer Positionsinformation eines in einem Eingriffsgebiet (39) eines Untersuchungsobjekts (5) befindlichen länglichen medizinischen Instruments (2), wobei innerhalb des medizinischen Instruments (2) wenigstens ein Lichtleiter (6) zu wenigstens einer distalen Messposition (36, 52), in der der Lichtleiter (6) zum Senden und Empfangen von Licht offenliegt, geführt ist, und ein extern zum Untersuchungsobjekt (5) angeordnetes Messgerät (9), das mit dem proximalen Ende des wenigstens einen Lichtleiters (6) verbunden ist, verwendet wird, aufweisend die Schritte:
- mittels einer Lichtquelle (8) des Messgeräts (9), Erzeugung von durch wenigstens einen des wenigstens einen Lichtleiters (6) zu der wenigstens einen Messposition (36, 52) zu führendem Sendelicht eines Sendespektrums,
- mittels eines Lichtdetektors (10) des Messgeräts (9), Empfang von Empfangslicht, das von der wenigstens einen Messposition (36, 52) über wenigstens einen des wenigstens einen Lichtleiters (6) zu dem Messgerät (9) geführt ist,
- mittels einer Auswertungseinheit des Messgeräts, Auswertung von das Empfangsspektrum des Empfangslichts beschreibenden Messdaten in Kenntnis des Sendespektrums zur Ermittlung einer die Position des medizinischen Instruments (2) in dem Untersuchungsobjekt (5) beschreibenden Positionsinformation auf der Grundlage von Reflektionseigenschaften von Materialien, insbesondere Geweben, des Untersuchungsobjekts (5), und
- Ausgabe der Positionsinformation an einer Darstellungseinrichtung (17).
